(19) Europäisches Patentamt

European Patent Office

Office européen des brevets

(11) EP 0 801 534 B1

(12) EUROPEAN PATENT SPECIFICATION

(45) Date of publication and mention
of the grant of the patent:
**10.12.2003 Bulletin 2003/50**

(51) Int Cl.⁷: $A61B\ 3/12$, $A61B\ 3/14$, $A61B\ 6/00$

(21) Application number: **94931769.7**

(22) Date of filing: **26.09.1994**

(86) International application number:
**PCT/US94/10877**

(87) International publication number:
**WO 96/009792 (04.04.1996 Gazette 1996/15)**

(54) **IMPROVED VISUALIZATION OF CHOROIDAL BLOOD FLOW AND ABERRANT VASCULAR STRUCTURES IN THE EYE**

OPTISCHE DARSTELLUNG DES BLUTKREISLAUFS UND VON ABNORMALEN GEFÄSSSTRUKTUREN IN DER ADERHAUT DES AUGES

VISUALISATION AMELIOREE DE LA CIRCULATION SANGUINE CHOROIDIENNE ET DES STRUCTURES VASCULAIRES ABERRANTES DANS L' OEIL

(84) Designated Contracting States:
**AT BE CH DE DK ES FR GB GR IE IT LI LU MC NL PT SE**

(43) Date of publication of application:
**22.10.1997 Bulletin 1997/43**

(60) Divisional application:
**00250386.0 / 1 084 675**
**00250385.2 / 1 084 674**

(73) Proprietor: **THE JOHNS HOPKINS UNIVERSITY**
**Laurel, MD 20723-6099 (US)**

(72) Inventor: **FLOWER, Robert, Walter**
**Hunt Valley, MD 21030 (US)**

(74) Representative: **Patentanwälte Wenzel & Kalkoff**
**Grubesallee 26**
**22143 Hamburg (DE)**

(56) References cited:
**WO-A-93/06770**          **US-A- 5 090 416**
**US-A- 5 150 292**          **US-A- 5 279 298**

## Description

## Field of the Invention

The invention relates to a method for improving the visualization of choroidal neovascularization (CNV) during angiography by injecting intravenously a fluorescent dye, the dye filling the vasculature of an eye; exciting the dye thereby causing a fluorescence to be emitted from the vasculature; and taking an angiographic image of the fluorescence from the CNV. Also, the invention relates to a device for bringing about such improved visualization comprising a fundus camera; an excitation light source coupled to the fundus camera; and a means for receiving images from the fundus camera.

## Prior Art

A related method and device are shown in US Patent 5,279,298 which aims at allowing a neovascular membrane in the ocular vasculature of the fundus of the eye to be identified and treated by eliminating the defect while damage to the sensory retina be minimized.

In general, it has to be emphasized that there is very little information about the blood flow through capillary plexuses which occurs on the time scale of the cardiac cycle. In part this is because direct visualization of such plexuses usually is technologically difficult or impossible, and most blood flow measurement methodologies require that data be obtained over many cardiac cycles. Moreover, when the capillary plexuses have complex vascular geometries and are fed by many arterioles, the additional problem of sorting-out blood flow distributions arises. One example of a capillary plexus is that found in the cerebral cortex. Another example, of great interest to scientists studying the eye, is the choriocapillaris, one of three blood vessel layers of the choroid.

The choroidal circulation of the eye bears a major responsibility for maintaining the sensory retina which lies above it. A prior art method has made possible routine visualization of the entire choroidal circulation, that is, all three vessel layers of the choroid can be visualized, superimposed one above the other. The innermost layer, the choriocapillaris, constitutes all of the nutritive vessels (i.e., where metabolic exchange with the retina takes place) for the choroidal circulation. The choriocapillaris layer occupies the plane immediately adjacent to the sensory retina.

Although choroidal angiograms show all of the vessels of the choroid, information pertaining specifically to the choriocapillaris is the most important, and there are conflicting views about the organization of the posterior pole choriocapillaris, particularly concerning blood flow through it. The method of extracting information about the choriocapillaris from an indocyanine green (ICG) angiogram is therefore an important one to the clinician who is interested in evaluating the metabolic sufficiency and stability of the choroidal circulation.

Numerous investigators have used angiography and a variety of histological techniques to collect the current body of information about the choroidal circulation. Although the gross aspects of choroidal angioarchitecture and blood flow have been amply revealed by investigators' efforts, controversies still exist regarding regional differences in morphology. Additional controversies have also arisen regarding details of blood flow through this highly complicated vascular network.

Of particular interest is blood flow through the choriocapillaris, since, as discussed above, it is in this vascular layer that the nutritive function of the choroidal circulation takes place. Even though the state of the larger choroidal blood vessels must certainly influence choriocapillaris blood flow, ultimately it is a precise understanding of the choriocapillaris blood flow itself that is fundamental to understanding the choroid's role in the pathophysiology of retinal disease.

High-speed indocyanine green (ICG) dye fluorescence angiography was developed to overcome the major problems encountered when attempting to visualize the rapid choroidal blood flow encountered in sodium fluorescein angiography. ICG angiography utilizes near-infrared wavelengths which penetrate the retinal pigment epithelium and choroidal pigment with relative ease. Whereas fluorescence from the choriocapillaris resulting from intravenously injected sodium fluorescein dye (the other standard dye used in ocular angiography) appears to arise mainly from extravasated dye molecules or those adhering to the vessel walls, ICG fluorescence arises from dye molecules bound to blood protein in the moving blood volume.

No doubt scanning laser ophthalmoscope fluorescein angiography (which can also utilize ICG dye) and the experimental technique of injecting fluorescein encapsulated in lipid vesicles eventually will produce additional information about choroidal blood flow; but with respect to clinical choroidal angiography, ICG angiography provides the best temporal and spatial resolution, making visualization of dye passage through the choroid possible under normal physiological conditions (i.e., without having to artificially slow blood flow by such methods as raising intraocular pressure).

When making intravenous dye injections, however, it is difficult to observe the choriocapillaris in individual ICG angiogram images due to the much higher levels of fluorescence arising from the large diameter underlying vessels. Due to this multi-layered organization of the choroidal vasculature, observation of the choriocapillaris with fluorescent dye angiography is best accomplished when a very small volume dye bolus having a sharply defined wavefront passes through. For example, following intra-carotid injection of a very small ICG dye bolus, ICG angiograms have been produced which clearly show the complete cycle of dye passage through an individual lobule under normal physiologic conditions. (Lobule is a term used to denote the three- to six-sided vascular units which form a mosaic pattern throughout the choriocapillaris. Each lobule consists of a cluster of narrow, tightly meshed capillaries which appear to radiate from a central focus at which a feeding arteriole enters at the posterior wall of the capillaries.)

Obviously, progression of a sharply defined wavefront is more easily tracked through the capillary network than an ill-defined one. Furthermore, if the bolus volume is small enough to essentially clear the underlying vascular layers by the time it enters the choriocapillaris, then images of the dye-filled capillaries will be of higher contrast than when significant fluorescence from beneath is simultaneously present.

Unfortunately, neither of the above conditions is readily produced by intravenous injection, even though passage of a dye bolus through the choroid can be optimized by appropriate injection technique. As a consequence, it is extremely difficult to isolate choriocapillaris dye filling in raw ICG fluorescence angiograms even when they are recorded at high speed. Therefore, there is a need for a method that will make it possible to extract information about choriocapillaris filling from venous-injection ICG dye angiograms.

Despite their inability to provide complete information about the choriocapillaris, ICG fluorescence angiograms of the choroidal circulation can delineate aberrant vascular structures in the choroid which significantly diminish vision. Age-Related Macular Degeneration (ARMD) is the leading cause of significant visual impairment in the elderly. This disease is frequently characterized by development of choroidal neovascularization (CNV) membranes which invade the sub-retinal space, resulting in displacement of the sensory retina, and often blocking of the visual pathway as a result of subsequent hemorrhage.

Treatment of ARMD is primarily by laser photocoagulation of the neovascular membrane. This treatment, however, is successful to the extent that the membrane can be accurately mapped; this is because such membranes are (by definition) in the macular area and often encroach on the fovea. Inappropriate application of photocoagulation can easily result in destruction of high acuity vision, and/or in accelerated growth of the CNV.

Diagnosis of and treatment of ARMD rely heavily upon interpretation of angiograms (both fluorescein and ICG). Frequently, the morphology of CNV lesions is such that the membranes appear in fluorescein angiograms as little more than fuzzy blurs, if at all, especially when the membrane lies beneath a serous detachment. Moreover, today it is recognized that for a class of CNV, referred to as "occult-CNV", ICG angiograms provide necessary treatment data which sodium fluorescein angiograms cannot.

A further major difficulty in utilizing ICG angiograms when applying laser photocoagulation therapy is that the retinal vascular landmarks upon which the surgeon must depend when aiming the laser are often missing from the ICG angiograms. The usual approach to resolving this problem is to make, during a separate setting, color photographs of the fundus and sodium fluorescein angiograms of the same eye of the patient; it is then necessary to attempt to superimpose the choroidal ICG angiogram and the retinal photograph or retinal fluorescein angiogram. This technique often fails due to the inability to precisely align the eye in exactly the same manner during each of the two angiographic procedures. Nevertheless, very accurate alignment (within as little as 50 microns on the retina) is vital to safely apply laser photocoagulation near the fovea and, at the same time, assure no significant permanent damage to the fovea itself.

Therefore, there exists a need for new methods and devices to permit both better visualization of aberrant vascular structures such as CNV and safer and more accurate laser photocoagulation to rid the eye of such structures and improve vision.

SUMMARY OF THE INVENTION

A method of visualisation of CNV (not claimed) is based on the premises that dye-filling of the choriocapillaris is more rapid --being pulsatile-- than dye-filling of the underlying larger diameter vessels and that fluorescence from these two overlapping layers is additive. The premise regarding the velocity of blood in the choriocapillaris runs contrary to conventional wisdom regarding the relationship between blood velocities in parent and daughter vessels in most vascular beds.

In a nutshell, the idea consists of recognizing that pixel-by-pixel subtraction of an image from a succeeding image in an ICG angiographic sequence of choroidal images forms a resultant image sequence which shows fluorescence arising only from structures where the most rapid movement of blood occurs, i.e., in the choriocapillaris and CNV vessels.

This subtraction enhancement method makes it possible to extract information about choriocapillaris dye filling by taking advantage of the differences in large vessel and choriocapillaris blood flow rates which naturally exist. Instead of distinguishing choroidal layers by temporal sequence of dye bolus appearance, it is dye filling rates which serve to separate them.

Implementation of the present method depends only upon configuring an existing fundus camera system to have sufficient temporal resolution and magnification of fundus structure. The described method was applied to high-speed ICG fluorescence angiograms to emphasize information about choriocapillaris hemodynamics.

In order to better visualize CNV and facilitate treatment of ARMD, however, the invention consists of a modified fundus camera with a polarizing filter in front of the excitation light source and an analyzing polarizer in front of the video camera. ICG dye fluorescence emanating from the fundus of the eye includes a significant component of polarized light, and rotation of the analyzer filter results in unwanted fluorescence (i.e., that not associated with vascular structures, but rather associated with scattered light) being suppressed to the extent that the underlying CNV can be better visualized. This particular process affects the unprocessed, raw angiographic images in that it improves the signal-to-noise content of the individual angiographic images; subsequently, the subtracted raw images result in a clearer resultant image.

More specifically, the present invention resides in providing the visualization method of the type comprising the steps set out in the initial paragraph of this specification by suppressing fluorescence other that fluorescence from the CNV using a polarizing filter in front of the excitation light source and an analyzing polarizer placed in a fundus camera when taking the angiographic image. The present invention also provides for a device for improving the visualization of CNV comprising a structure of the type also explained in said initial paragraph of this specification by placing a polarizing filter in front of the light source and an analysing polariser between the receiving means and the fundus camera, with the receiving means optionally comprising a video camera.

Once the aberrant vascular structure has been visualized and delineated by the polarization and subtraction methods but before laser photocoagulation therapy can begin, the surgeon must be assured that he or she can properly aim the laser. The present method may further rely on the usual practice of performing fluorescein angiography prior to performing ICG angiography and makes use of the fact that the fluorescein dye remains associated with the retinal vasculature for more than one hour.

There may be utilized an ICG fundus camera which has an integrating sphere coupled to light sources for excitation of both ICG and sodium fluorescein dye fluorescences and which uses a gated charge-coupled device (CCD) video camera to capture the angiographic images. Light input to the integrating sphere is via two fiber optic cables, each connected to one of two light sources. One source is laser output at the wavelength needed to excite sodium fluorescein dye (480nm, i.e., a frequency-doubled Nd-Yag); it is also recognized that a shuttered, filtered incandescent light source can be used in place of a frequency-doubled laser. The other source is a diode laser output for excitation of ICG dye (805nm).

As ICG dye transits through the choroidal circulation, the gated video camera records images of the ICG dye by causing the 805nm laser diode to fire in synchrony with the video camera. Appropriate programming of the camera and light sources are configured such that at regular intervals (e.g., every eighth image) the 480nm light source is fired and, simultaneously, an appropriate change is made in the barrier filter in front of the video camera.

To use the every-eighth-frame example, a barrier filter change is implemented simply by placing a rotating disk containing eight filters in front of the video camera. This filter wheel turns in synchrony with the camera firings such that every eighth frame corresponds to a positioning of the sodium fluorescein barrier filter in front of the camera. Because the sequence of angiograms is made at high speeds (approximately 15-30 images/second), eye movements between successive images is insignificant, making precise registration of images trivial. Thus, the method provides the ability to precisely superimpose the retinal vessel landmarks contained in sodium fluorescein angiograms on the delineated CNV lesions in the ICG angiograms, as needed by the surgeon to accurately focus a laser for treatment.

BRIEF DESCRIPTION OF THE DRAWINGS

Fig. 1, consisting of Figs. 1a and 1b, illustrates an ICG fluorescence image of layers of ICG-stained blood to demonstrate fluorescence additivity and a graph produced from the image, respectively.

Fig. 2, consisting of Figs. 2a and 2b, illustrates schematically the brightness of fluorescent light emitted by two different blood vessels at times $t_1$ and $t_2$, respectively.

Fig. 3, consisting of Figs. 3a, 3b, 3c and 3d, are, in 3a and 3b, ICG fluorescence images showing a 50 degree field of view centered on the macula of a right eye; the images were made 1/15 second apart. Fig. 3c is the result of subtracting the image of Fig. 3a from the image of Fig. 3b, and Fig. 3d is simply an enlargement of Fig. 3c.

Fig. 4 illustrates a fundus camera system modified to provide the angiograms seen in Figs. 3a and 3b.

Fig. 5, consisting of Figs. 5a, 5b, 5c and 5d, illustrates four images of a left eye selected from a sequence of images produced by the subtraction method of the invention.

Fig. 6 illustrates a fundus camera system modified to suppress unwanted fluorescence.

Fig. 7 illustrates a fundus camera system modified to provide superimposed angiograms.

DETAILED DESCRIPTION OF THE PREFERRED EMBODIMENT

Repeated real-time observations have shown that during ICG dye transit, after the large choroidal arteries fill, there is a rapidly pulsating faint and diffuse fluorescence superimposed over the steady fluorescence of the large vessels at the posterior pole. These pulsations appear to occur at a greater frequency than the heart rate, and they appear less obvious by the time the large choroidal veins are filled. Subsequent frame-by-frame analysis of the angiograms, however, indicate that the greater-than-heart-rate frequency is a perceptual phenomenon resulting from the out-of-phase pulsatile filling of individual lobules, all at near-heart-rate frequency.

Unfortunately, not enough is known yet about details of choriocapillaris hemodynamics to account with certainty for the observed more rapid fluorescence intensity changes in the choriocapillaris than in the larger underlying vessels, but the most likely reason is that choriocapillaris blood flow velocity is greater than that through the underlying choroidal vessels. The invention is based on the premises that the fluorescence intensities of ICG-filled choriocapillaris and underlying vessels are additive and that there are detectable differences in the rates of change of fluorescence intensities emanating from the choriocapillaries and the underlying choroidal vessels as they fill with dye.

Although the average cross-sectional diameter of the choriocapillaris is much smaller than that of the underlying arterial and venous vessels which feed and drain them, it appears that fluorescence from the two vascular layers is additive. ICG fluorescence additivity was demonstrated by creating a stair-step wedge of overlapping thin layers of heparinized blood containing ICG dye (0.03 mg/ml); each step was formed by a thin layer of the blood sandwiched between two microscope slide coverglasses.

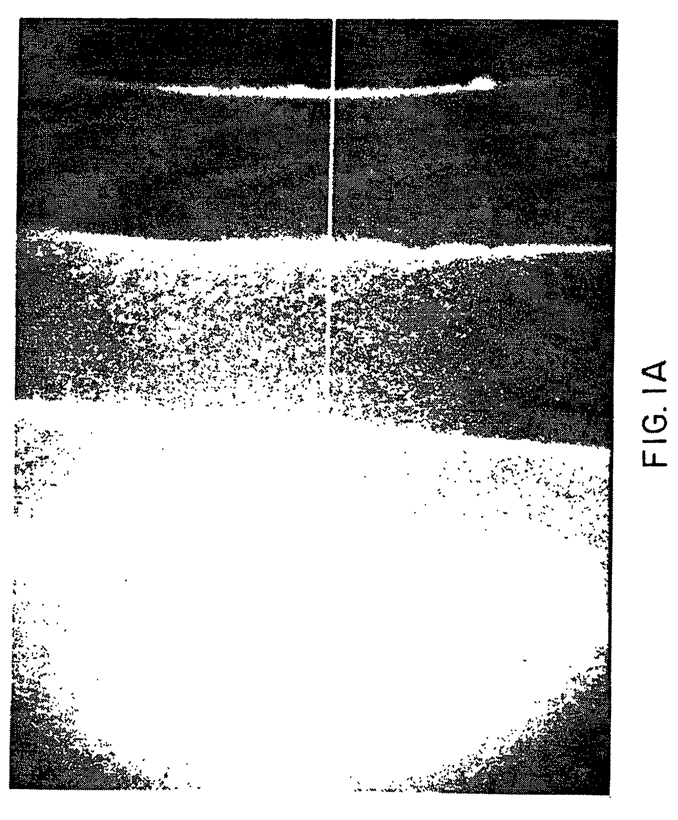

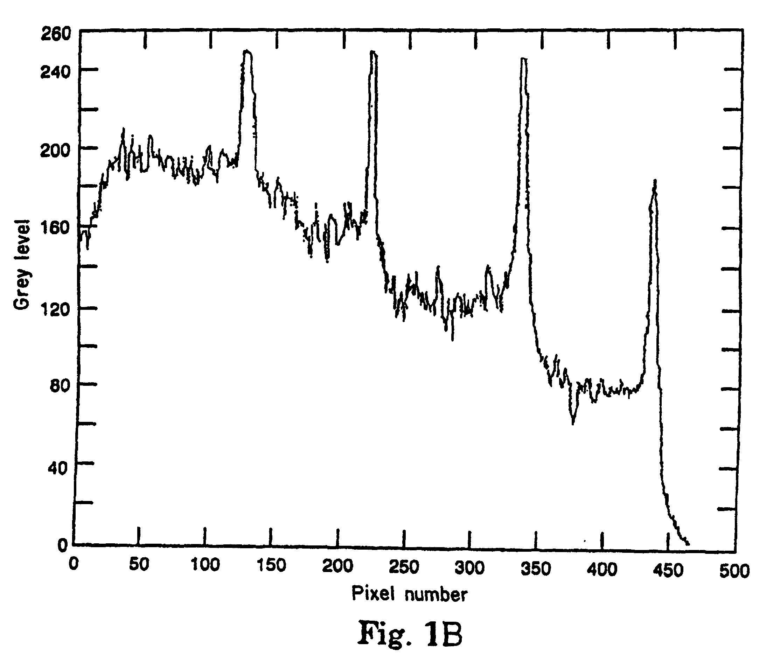

Fig. 1a shows an ICG fluorescence image of the stair steps. The horizontal white line through the center of the image indicates the path along which image pixel brightness (i.e., grey level) was measured to produce the graph in Fig. 1b, demonstrating stepwise increase in fluorescence as the number of overlapping blood layers increased.

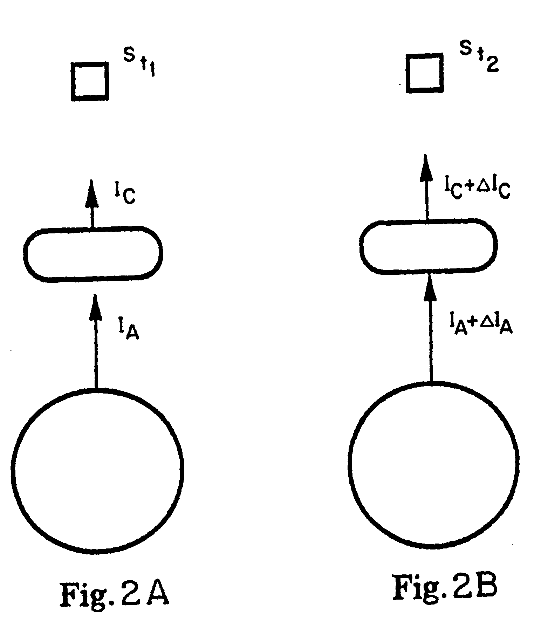

The greater rate of change in dye fluorescence intensity in choriocapillaries than in the larger underlying vessels is shown schematically in Figs. 2a and 2b. In Fig. 2a, the brightness of a large diameter vessel and an overlying choriocapillaris vessel (both in cross-section) are indicated as vectors, $I_A$ and $I_C$, respectively. The fluorescent light emitted by both is detected at time $t_1$ by a light sensor, S. In Fig. 2b, the status of the same two vessels and sensor is shown at later time $t_2$, where $\Delta I_A$ and $\Delta I_C$ are respectively the incremental increases in brightness of the two vessels. Therefore, the total brightness detected by the sensor at $t_1$ is:

$$S_{t1} = I_A + I_C$$

At time $t_2$, the total brightness detected is:

$$S_{t2} = I_A + I_C + \Delta I_A + \Delta I_C$$

The change in total detected brightness which occurred between $t_1$ and $t_2$, $\Delta S$, then is:

$$\Delta S = S_{t2} - S_{t1} = \Delta I_A + \Delta I_C$$

But since $\Delta I_A << \Delta I_C$, $\Delta S = \Delta I_C$.

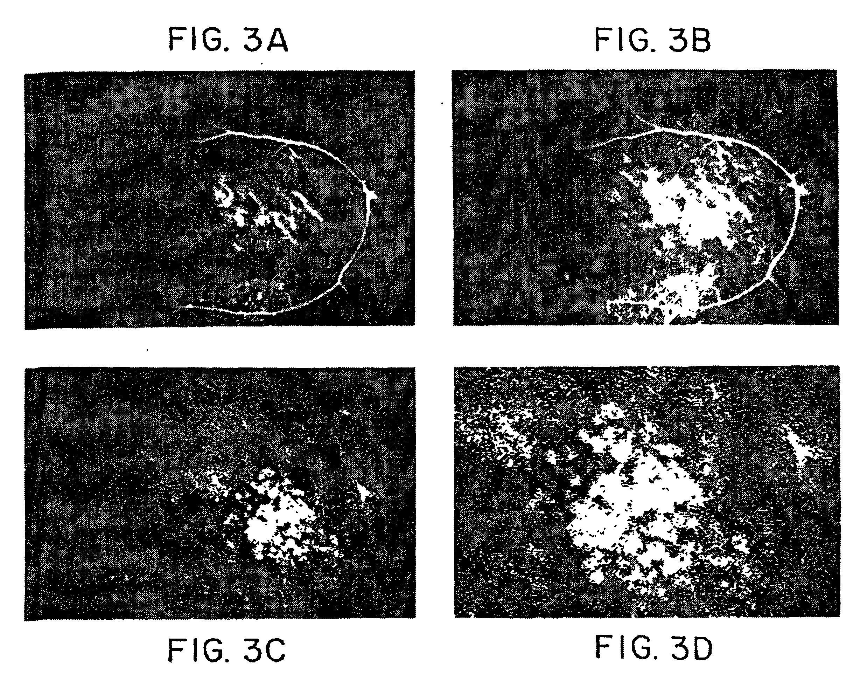

In other words, the small change in the combined brightness of the overlapping capillary and large vessel which occurs during a short time interval is virtually all attributable to the choriocapillaris vessel. This phenomenon can be demonstrated by the method of the invention, i.e., by subtracting, pixel for pixel, an image in a high-speed ICG fluorescence angiogram sequence from a succeeding image, as demonstrated in Figs. 3a-d. Figs. 3a and 3b are angiographic images made 1/15 second apart. Fig. 3c is the result of subtracting those two images, and Fig. 3d is simply an enlargement of Fig. 3c.

Note that in the resultant image (Fig. 3c or 3d) lobular structures are seen which were not apparent in either of the original images (Fig. 3a or 3b). Also, instead of the dye-filled retinal arteries seen in the original images, only a dye wavefront representing the movement of additional dye into the retinal arteries near the disc is seen in the resultant image. Of course, the more spatially well defined the dye bolus, the more dramatic is the effect of the invention. Not all intravenously injected dye boluses produce as dramatic results as were achieved in this example, but in each case there is enhancement of the choriocapillaris component of fluorescence. Note, the subtraction method of the invention is intended to operate by subtracting the image from any succeeding image.

To test the method, five normal rhesus monkeys between two and three years of age were used. For each observation a monkey was immobilized by intramuscular injection of ketamine hydrochloride (10 to 15 mg/kg), intubated, and then maintained lightly anesthetized with halothane; mydriasis was induced by topical application of 1% tropicmide. Small boluses (about 0.05 ml) of ICG dye (12.5 mg/ml) were injected through a catheter inserted in the greater saphenous vein and immediately followed by a 2.0 ml saline flush. Passage of dye through the choroidal vasculature was detected using a modified Zeiss fundus camera and directly digitally recorded by PC-based video frame-grabbers. At least three angiographic studies of the same eye were performed on different days for each monkey.

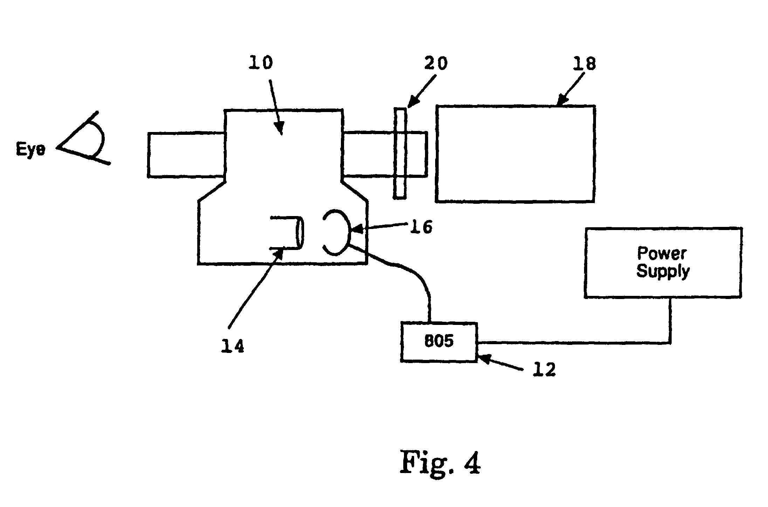

In the above test, as shown in Fig. 4, the usual fundus camera 10 was modified by replacing the xenon flash tube light source with an 805 nm wavelength laser diode 12 coupled to the fundus camera's illumination optics 14 via a small integrating sphere 16 whose exit port was located at the position normally occupied by the flash tube arc. The fundus camera's usual means for receiving images, i.e., the photographic film camera, was replaced with an infrared sensitive vidicon tube (model 4532URI Ultracon, Burle Industries) 18 (a charge-coupled device could be used instead of the vidicon tube), in front of which an 807 nm wavelength cut-on filter 20 was placed to exclude the excitation laser light while admitting ICG dye fluorescence light. Choroidal dye transit was recorded in thirty-two consecutive video angiographic images at a rate of 30 or 15 frames per second by two digital frame grabbers (model 2861-60, Data Translation) (not shown) installed in a personal computer (Compaq, model 386/25e) (not shown).

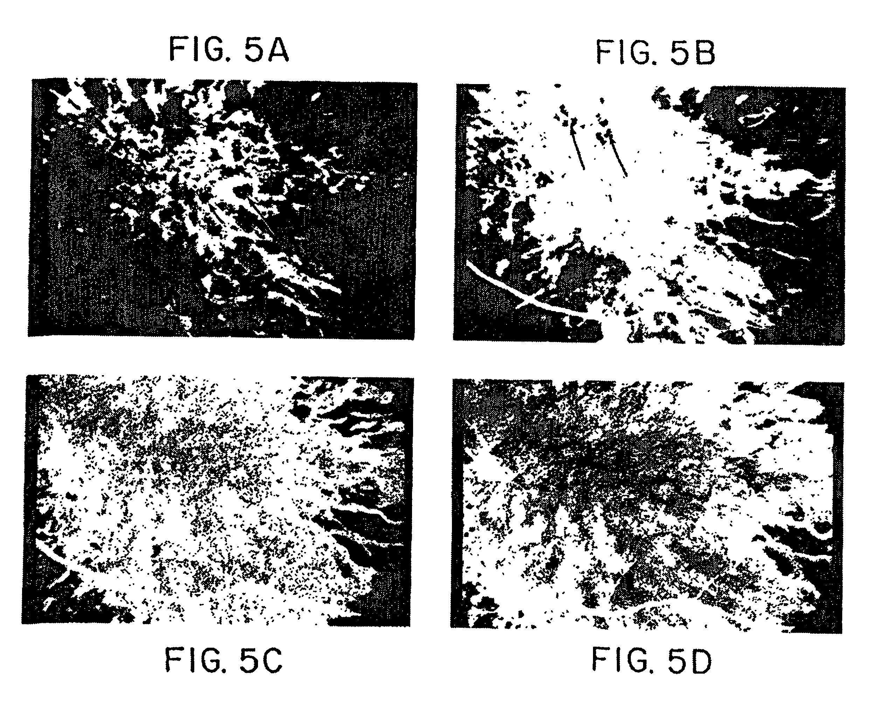

Fig. 5 summarizes the angiographic findings obtained in the above test by applying the image subtraction method of the invention. In this example case, each image in a 15 frames/second ICG angiographic sequence was subtracted from the image immediately following it; the images in Fig. 5 were selected from the resulting sequence of subtracted images.

Dye first enters the macular area of the choriocapillaris which lies temporal to and above the points at which the short posterior ciliary arteries enter the eye (Fig. 5a). A lobular pattern can be seen in the center of the angiogram, particularly just nasal to the center; here a cluster of unfilled lobules is shown (arrows). 0.133 seconds later (Fig. 5b) the entire central area is completely filled, although two smaller clusters of late-filling lobules may be seen superior to the center (arrows). Choriocapillaris filling progresses almost radially from the macular region. By close inspection of this image, faint loss of fluorescence around lobules can be seen; these likely correspond to choriocapillaris drainage channels.

Fig. 5c is 0.200 seconds later than Fig. 5b. It indicates that the radially oriented wave of choriocapillaris dye filling has been completed, and dye distribution at the posterior pole region appears fairly uniform. This image indicates that the first wave of dye filling is complete within the center of the macular region, as indicated by the appearance of relatively hypo-fluorescent areas which were hyper-fluorescent in Fig. 5a.

In Fig. 5d, 0.133 seconds later, it appears that the first wavefront of dye filling has reached the peripheral region; at this stage, Fig. 5d is nearly a complete reverse contrast image of Fig. 5a.

The wavefront of dye filling traveled radially from the macular region to the periphery of the 30 degree field of view in approximately 0.466 seconds. This overall filling pattern was present in each eye observed, and details of the filling patterns were remarkably consistent from observation to observation for each subject eye.

ICG fluorescence angiography gradually is being used more frequently by both researchers and clinicians to investigate the choroidal circulation. Clearly, as such new tools are applied in a variety of new ways to studying the choroid, old concepts about it and its physiology will be revisited, and some will change or give way to entirely new concepts. Fortunately, some approaches to analyzing choroidal angiograms like the subtraction method described above may be applied both in animal and in human clinical research with complete safety, perhaps hastening a better understanding of choroidal blood flow in health and disease.

ICG fluorescence angiography is used in the diagnosis and treatment of ARMD; however, as noted above, the difficulty arises in attempting to accurately map choroidal neovascularization (CNV). One novel aspect of this method lies in recognizing that fluorescence arising from a dye molecule contains information about the processes that take place within the molecule during the time between excitation and emission of light by the molecule. Moreover, fluorescence of molecules can be affected by the characteristics of the substances to which the molecule is bound and by the character of the binding which has taken place.

For example, in the case of ICG dye in the vasculature of an eye containing CNV, the dye may bind with greater affinity to neovascular endothelium than to established endothelium. In such a case, fluorescence arising from those bound dye molecules may be substantially different from fluorescence associated with ICG dye molecules which may be bound to other types of protein in the cirrus fluid or from ICG fluorescent light simply scattered by the presence of protein molecules within the cirrus fluid. In either event, ellipsometry is an appropriate tool for improving the visualization of CNV.

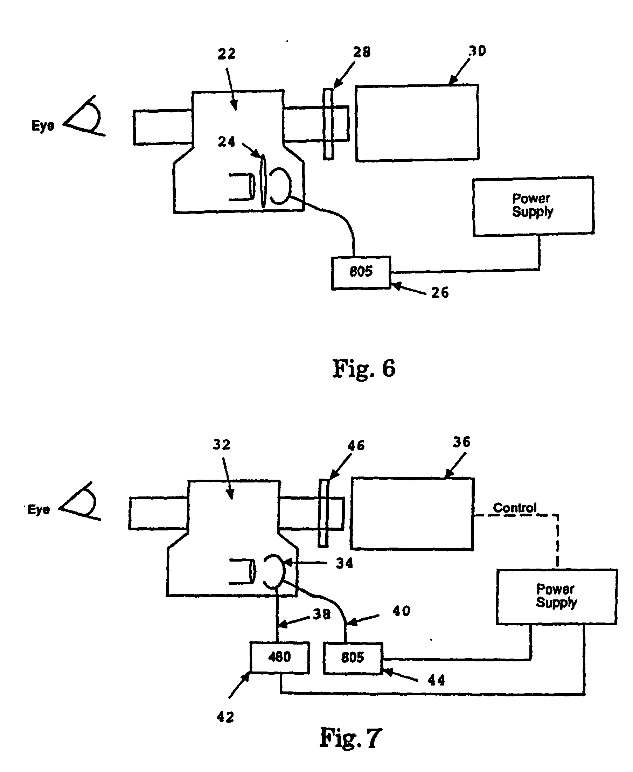

The method then, as shown in Fig. 6, is a modified fundus camera 22 with a polarizing filter 24 in front of the excitation light source 26 and an analyzing polarizer 28 in front of the video camera 30. ICG dye produces a high degree of polarized light, and rotation of the analyzer filter results in the fluorescence from the serous fluid being suppressed to the extent that the underlying CNV can be better visualized. This particular process affects the unprocessed, raw angiographic images in that it improves the signal-to-noise content of the individual angiographic images; subsequently, the subtracted raw images result in a clearer resultant image.

Once an aberrant vascular structure such as CNV is clearly delineated, it can be treated using laser photocoagulation therapy; however, as noted above, aiming the laser properly requires superimposing an ICG angiogram and a retinal photograph or retinal fluorescein angiogram. The method results from the usual practice of performing fluorescein angiography prior to performing ICG angiography making use of the fact that the fluorescein dye remains associated with the retinal vasculature for quite long periods of time (more than one hour). Therefore, if one configures an ICG fundus camera in such a way that during the course of obtaining ICG angiograms, a fluorescein angiogram can be obtained (within fractions of a second of obtaining a previous and succeeding ICG angiogram), no significant movement of the eye can take place. This means that the intervening fluorescein angiogram would, by definition, precisely register with the ICG angiograms.

As shown in Fig. 7, the device utilizes an ICG fundus camera 32 which has an integrating sphere 34 coupled to light sources for excitation of ICG dye fluorescence and which uses, as an image receiving means, a gated video camera 36 (preferably CCD) to capture the angiographic images. Light input to the integrating sphere is via two fiber optic cables 38, 40, each connected to one of two light sources 42, 44; one source 42 output is at the wavelength needed to excite sodium fluorescein dye (480nm) and the other source 44 output for excitation of ICG dye (805nm).

As ICG dye transits through the choroidal circulation, the gated video camera 36 records images of the ICG dye by causing the 805nm laser source 44 to fire in synchrony with the video camera 36. Appropriate programming of the camera and light sources are configured such that at regular intervals (e.g., every eighth image) the 480nm source 42 is fired, and simultaneously an appropriate change is made in the barrier filter 46 in front of the video camera.

To use the every-eighth frame example, the barrier filter chain is implemented simply by placing a rotating disk containing eight filters in front of the video camera. This filter wheel turns in synchrony with the camera firings such that every eighth frame corresponds to a positioning of the fluorescein barrier filter in front of the camera. Thus, the device provides the ability to precisely superimpose angiograms needed by the surgeon in order to accurately aim a laser photocoagulation beam.

**Claims**

1. A method for improving the visualization of choroidal neovascularization (CNV) during angiography comprising the steps of:

   - injecting intravenously a fluorescent dye, the dye filling the vasculature of an eye;

   - exciting the dye by light from a light source thereby causing a fluorescence to be emitted from the vasculature; and

   - acquiring an angiographic image of the fluorescence from the CNV by a fundus camera;

   **characterized by**:

   - suppressing fluorescence other than fluorescence from the CNV using a polarizing filter in front of the light source and an analyzing polarizer in the fundus camera when taking the angiographic image.

2. A device for improving the visualization of choroidal neovascularization (CNV) comprising:

   - a fundus camera (22);

   - an excitation light source (26) synchronized to the fundus camera; and

   - a means (30) for receiving images from the fundus camera;

   **characterized by**:

   - a polarizing filter (24) placed in front of the light source; and
   - an analyzing polarizer (28) placed between the receiving means and the fundus camera.

3. The device as recited in claim 2, wherein the receiving means comprises a video camera (30).

**Patentansprüche**

1. Ein Verfahren zum Verbessern der Sichtbarmachung chorioidaler Neovaskularisation (CNV) während der Angiographie, umfassend die Schritte:

   - intravenöses Injizieren eines fluoreszenten Farbstoffes, wobei dieser Farbstoff die Gefäßanordnung (Vaskulatur) des Auges füllt;
   - Anregen des Farbstoffes durch Licht von einer Lichtquelle, wodurch ein Ausstrahlen von Fluoreszenz aus der Gefäßanordnung hervorgerufen wird; und
   - Gewinnen eines angiographischen Bildes der Fluoreszenz von der CNV durch eine Funduskamera;

   **gekennzeichnet durch**

   - Unterdrücken von Fluoreszenz außer der Fluoreszenz von der CNV unter Verwendung eines polarisierenden Filters vor der Lichtquelle und eines analysierenden Polarisators in der Funduskamera beim Aufnehmen des angiographischen Bildes.

2. Eine Einrichtung zum Verbessern der Sichtbarmachung chorioidaler Neovaskularisation (CNV), umfassend:

   - eine Funduskamera (22);
   - eine Anregungslichtquelle (26), die mit der Funduskamera synchronisiert ist; und
   - ein Mittel (30) zum Empfangen von Bildern von der Funduskamera;

   **gekennzeichnet durch**

   - ein Polarisationsfilter (24), das vor der Lichtquelle angeordnet ist; und
   - einen analysierenden Polarisator (28), der zwischen dem Empfangsmittel und der Funduskamera angeordnet ist.

3. Die Einrichtung nach Anspruch 2, wobei das Empfangsmittel eine Videokamera (30) umfaßt.

**Revendications**

1. Procédé destiné à améliorer la visualisation de la néovascularisation choroïdale (CNV) pendant l'angiographie comprenant les étapes consistant à :

   -- injecter par voie intraveineuse une substance colorante fluorescente, la substance colorante remplissant la vascularisation d'un oeil ;
   -- exciter la substance colorante par de la lumière provenant d'une source lumineuse provoquant ainsi l'émission d'une fluorescence émise par la vascularisation; et
   -- acquérir une image angiographique de la fluorescence provenant de la CNV par une caméra de fond ;

   **caractérisé en ce que**

   -- on supprime la fluorescence autre que la fluorescence provenant de la CNV en utilisant un filtre polarisant en avant de la source lumineuse et un polariseur d'analyse dans la caméra de fond lorsqu'on prend l'image angiographique.

2. Dispositif destiné à améliorer la visualisation de la néovascularisation choroïdale (CNV) comprenant :

   -- une caméra de fond (22) ;
   -- une source lumineuse d'excitation (26) synchronisée avec la caméra de fond ; et
   -- un moyen (30) pour recevoir des images provenant de la caméra de fond ;

   **caractérisé par** :

   -- un filtre polarisant (24) placé en avant de la source lumineuse ; et
   -- un polariseur d'analyse (28) placé entre le moyen de réception et la caméra de fond.

3. Dispositif selon la revendication 2, dans lequel les moyens de réception comprennent une caméra vidéo (30).

FIG. IA

Fig. 1B

Fig. 2 A          Fig. 2 B

FIG. 3A

FIG. 3B

FIG. 3C

FIG. 3D

EP 0 801 534 B1

Fig. 4

FIG. 5B

FIG. 5A

FIG. 5D

FIG. 5C

Fig. 6

Fig. 7